# EUROPÄISCHE PATENTSCHRIFT

(11) **EP 1 341 544 B1**
(45) Veröffentlichungstag und Bekanntmachung des Hinweises auf die Patenterteilung: **24.05.2006**
(21) Anmeldenummer: 01270335.1
(22) Anmeldetag: 11.12.2001
(51) Int. Cl.: A61K 31/70, A61P 31/00

(54) **BLOCKIERUNG DER ANLAGERUNG VON KEIMEN BEI VÖGELN**
BLOCKING THE ADSORPTION OF GERMS IN BIRDS
BLOCAGE DU DEPOT DE GERMES CHEZ LES OISEAUX

(30) Priorität: 11.12.2000 DE 10061574
(43) Veröffentlichungstag der Anmeldung: 10.09.2003
(73) Patentinhaber: de Bettignies-Dutz, Andreas, Dr., 1130 Wien (AT); Guggenbichler, Josef Peter, Prof. Dr., 90762 Fürth (DE); Jurenitsch, Johann, Prof. Dr., 1020 Wien (AT)
(72) Erfinder: GUGGENBICHLER, Josef, Peter, 80336 München (DE); JURENITSCH, Johann, A-1020 Wien (AT)
(74) Vertreter: Huber, Bernhard
(86) Internationale Anmeldenummer: PCT/EP2001/014541
(87) Internationale Veröffentlichungsnummer: WO 2002/047695

(56) Entgegenhaltungen:
- EP-A- 0 888 776
- WO-A-95/07084
- US-A- 3 316 242

## Beschreibung

Die vorliegende Erfindung betrifft die Verwendung eines pharmazeutischen Präparats, das als Wirkstoff ein Oligogalakturonid enthält zur Blockierung der Anlagerung von Keimen an Vogelzellen.

Es ist bekannt, dass die Adhärenz pathogener Keime über .spezifische Rezeptoren auf den Zelloberflächen erfolgt. Diese Rezeptoren haben sich in den verschiedenen Klassen des Tierreiches sehr unterschiedlich entwickelt. So weisen z.B. Proteine von Vogelzellen völlig andere Glykosylierungsmuster auf als beispielsweise Säuger- oder Reptilienzellen.

Aus der EP 0 716 605 ist es bekannt, dass Oligogalakutronide mit einem Polymerisationsgrad ≥ 2 und einem Veresterungsgrad kleiner als 20 % in der Lage sind, die Anlagerung von Keimen an Säugerzellen zu blockieren.

Säugerzellen weisen spezifische Oberflächenstrukturen auf, die Keimen generell und pathogenen Mikroorganismen im Speziellen die Anhaftung ermöglichen. Bei diesen spezifischen Strukturen handelt es sich in der Regel um Kohlehydratstrukturen, die Spezies-spezifisch sind. So wird beispielsweise die Haftung von E.coli an die Epithelzellen des Gastrointestinaltraktes durch bakterielle Lectine vermittelt, die Oligosaccharideinheiten auf der Oberfläche der Zielzellen erkennen. Die Kohlehydratstrukturen auf der Oberfläche, der Zellen werden daher als wesentlich angesehen für die bekannte Spezies-spezifische Pathogenität von Mikroorganismen. Überraschenderweise wurde nunmehr gefunden, dass die bei Säugerzellen gefundene Blockierung der Anhaftung von Keimen durch die genannten Oligogalakturonide auch die Anhaftung von Keimen an Vogelzellen zu blockieren vermag. Dies ist überraschenf, da Vogelzellen andere Oligosaccharidstrukturen auf ihrer Oberfläche tragen als Säugerzellen, vermutlich bedingt durch die schon sehr lange zurückliegende entwicklungsgeschichtliche Trennung zwischen der Entwicklung von Vögeln und Säugern.

Bei Vögeln, insbesondere bei der in Massentierhaltung, gehaltenen Geflügelzucht, stellen Infektionen durch Mikroorganismen ein großes Problem dar. Bisher konnten derartige Infektionen mit ausreichender Erfolgsschance nur durch die Verabreichung von Antibiotika bekämpft werden. Da aufgrund der weit verbreiteten Anwendung von Antibiotika gerade in der Tierzucht jedoch die Gefahr der Entwicklung von Antibiotikaresistenz bei den pathogenen Mikroorganismen bereits große Besorgnisse ausgelöst hat, besteht ein Bedürfnis, Antibiotika für diesen Einsatzzweck durch andere Mittel zu ersetzen. Dies wird noch dadurch verstärkt, dass in vielen Staaten die Verwendung von Antibiotika in der Tierzucht nicht mehr gestattet ist oder ein Verbot der Antibiotikaverwendung für diesen Zweck bevorsteht.

Da nun, wie oben erwähnt, insbesondere bei der Massengeflügelhaltund ein hohes Infektionsrisiko besteht, werden Arzneimittel dringend gebraucht, die eine derartige Blockade der Adhärenz von Keimen auch in Vogelzellen bewirken können.

Es besteht daher ein großes Bedürfnis nach der Schaffung eines Mittels, welches anstelle von Antibiotika bei Vögeln bei Mikroorganismeninfektionen eingesetzt werden kann und auch vorbeugend verwendet werden kann, um das Anhaften von pathogenen Keimen an die Zellen der Vögel zu beseitigen oder zu verhindern. Die Aufgabe der vorliegenden Erfindung besteht somit darin, ein Arzneimittel bereitzustellen, weiches die Anlagerung von Keimen an Vogelzellen, insbesondere an Vogelepithelzellen, blockiert.

Gelöst wird diese Aufgabe erfindungsgemäß durch ein Verfahren zur Herstellung eines pharmazeutischen Präparates zur Blockierung der Anlagerung von Keimen an Vogelzellen, welches dadurch gekennzeichnet ist, dass man ein oder mehrere Oligogalakturonide mit einem Polymerisationsgrad ≥ 2 und einem Veresterungsgrad < 20 % als Wirkstoff, gegebenenfalls zusammen mit pharmazeutisch üblichen Träger-, Verdünnungs-, Hilfs- und Füllstoffen in eine zur Verabreichung geeignete Form bringt.

Es ist bekannt; dass die Fähigkeit von Keimen zur Bildung von Toxinen, z.B. von Enterotoxinen durch E.coli einen wesentlichen Virulenzfaktor darstellt. Jedoch auch die Besiedlung von Schleimhäuten durch Keime, die durch die Adhärenz an Kohlenhydratrezeptoren des Epithels der Darmzotten bzw. des Urogenitalepithels mit Spezies-spezifischen Fimbrien ermöglicht wird, ist als initialer erster Schritt jeder Infektion für die Virulenz eines Keimes von wesentlicher Bedeutung. Untersuchungen der Pathogenese von Infektionen legen nahe, dass die Adhärenz der Keime an Epithelzellen und die dadurch vermittelte Besiedelung von Schleimhäuten der Bildung von Toxinen als Virulenzfaktor gleichwertig ist.

Einige dieser Rezeptor-analogen Kohlenhydrate wurden isoliert und deren Struktur aufgeklärt. In vitro Untersuchungen konnten eine vollständige Blockierung der Adhärenz zahlreicher Bakterienspezies wie EHEC, ETEC, EPEC, E. coli 078/K80, Salmonella spp. Klebsiella spp., Enterobacter spp. u.a. durch niedrige Konzentrationen dieser Kohlenhydrate nachweisen. Es war nun von Interesse, ob diese Kohlenhydrate, die in vitro eine hervorragende Blockierung von pathogenen Mikroorganismen bewirken, in einem therapeutischen Versuch im Vogelmodell zu einer Verminderung septischer und lokaler Komplikationen führen.

Überraschenderweise wurde gefunden, dass durch Karottensuppe in der klassischen Zubereitung nach MORO, Blasentee (z.B. eine Mischung aus Zinnkraut, gelbem Himmelschlüssel, Erdbeerbiättern und Isländischem Moos), Kokosmilch, Kranbeeren etc. die Adhärenz pathogener Keime, wie etwa E.coli, an Vogelzellen, insbesondere an Epithelzellen des Gastrointestinal- und Urogenitaltrakts wesentlich (d.h. bis zu 90 %) reduziert werden kann. Diese Wirkung ist auf die in den Pflanzenprodukten vorhandenen Pektine zurückzuführen, bei denen es sich im Wesentlichen aus Ketten von 1,4-α-glycosidisch verbundenen Galakturoniden handelt, deren Säuregruppen zu 20 bis 80 % mit Methanol verestert sind; und die neben Galacturonsäure gegebenenfalls noch andere Zuckerbausteine, z.B. Glucose, Galactose, Xylose und Arabinose enthalten können.

Überraschenderweise wurde festgestellt, dass Pektine bzw. Oligogalakturonide, die bisher zur Bildung von Gelees, als Verdickungsmittel oder als Ballaststoffe verwendet wurden, die Anlagerung von Keimen an Vogelzellen blockieren.

Ein zur Blockierung der Adhärenz von Keimen wirksames Oligogalakturonid muss einen bestimmten Polymerisationsgrad und vorzugsweise einen bestimmten Veresterungsgrad aufweisen. Der Begriff "Polymerisationsgrad" im Sinne der vorliegenden Erfindung bezeichnet die Anzahl der Galacturonsäureeinheiten, die ein Oligogalakturonid enthält. Der Begriff "Veresterungsgrad" bezeichnetdie Prozent(meistdurch Methyl) veresterten Galacturonsäureeinheiten eines Oligogalakturonids bezüglich der Gesamtzahl von Galacturonsäureeinheiten. Es wurde festgestellt, dass zur Blockierung der Adhärenz von Keimen an Vogelzellen ein Polymerisationsgrad ≥ 2 erforderlich ist, d.h. monomere Galacturonsäure zeigt - ebenso wie eine Vielzahl anderer Saccharide (z.B. neutrale Saccharide, Glucuronsäure, Arabinogalactan, Galactose-1-phosphat, Glucose-1-phosphat) - keine Blockierung der Adhärenz von Keimen.

Erfindungsgemäß bevorzugt ist die Verwendung von Oligogalakturoniden mit einem Veresterungsgrad < 20 %, besonders bevorzugt < 10 % und noch mehr bevorzugt < 5 % in dem erfindungsgemäßen pharmazeutischen Präparat. Am meisten bevorzugt sind im Wesentlichen keine (< 2 %) Estergruppen mehr vorhanden.

Oligogalakturohide mit einem Veresterungsgrad < 20 % sind aus natürlich vorkommenden Pektinen durch vollständige oder teilweise Demethylierung, z.B. durch Verseifung mit Alkali, erhältlich.

Der Polymerisationsgrad von Oligogalakturoniden in einem erfindungsgemäßen pharmazeutischen Präparat ist ≥ 2, d.h. das Oligogalakturonid weist mindestens zwei Galacturonsäureeinheiten auf. Vorzugsweise ist der Polymerisationsgrad des Öligogalakturonids 2 bis 7, noch mehr bevorzugt 2 bis 4, besonders bevorzugt 2 bis 3 und am meisten bevorzugt 2. Das heißt, Digalacturonid, Trigalacturonid, Tetragalacturonid, Pentagalactüronid, Hexagafacturonid, Heptagalacturonid und Mischungen von mehreren dieser Substanzen sind bevorzugte Oligogalakturonide für die erfindungsgemäßen Präparate, wobei Digalacturonid bzw. Digalacturonsäure am meisten bevorzugt ist. Abhängig von der Art der Anwendung können jedoch auch Präparate verwendet werden, die nicht die von der Wirkung her beste Substanz enthalten, wenn diese Substanz aus pharmazietechnischen Gründen für eine spezielle Anwendungsform weniger günstig ist.

Oligogalakturonide mit einem Polymerisationsgrad von 2 bis 4 sind aus natürlich vorkommenden, höher molekularen Pektinen durch enzymatische Hydrolyse unter Verwendung von Pektin-spaltenden Enzymen (z.B. Pektinasen) erhältlich, die in der Technik meist aus Pilzen, wie etwa Aspergillus- oder Penicilliumarten gewonnen werden. Andererseits kann die Hydrolyse der höher molekularen Pektine auch durch saure Hydrolyse z.B. mit HCl erfolgen.

Die erfindungsgemäßen pharmazeutischen Präparate können die Anlagerung von Keimen, insbesendere von bakteriellen Keimen (z.B. E.coli, Streptokokken, Salmonella spp., Klebsiella spp., Enterobacter spp., Haemophilus influenza, Pneumokokken etc.) an Vogelzellen blockieren bzw. bereits gebundene Keime verdrängen. Insbesondere eignen sich die erfindungsgemäßen Präparate zur Behandlung von Infektionen des Gastrointestinaltrakts, des Blutsystems (hämolytisches urämisches Syndrom, durch E.coli 0157 verursacht), der Atemwege, des Urogenitaltrakts (z.B. rezividierende Infektionen des Harntrakts) oder/und des Rachenraums (z.B. durch Streptokokken, H. influenzae, Pneumokokken).

Die erfindungsgemäßen pharmazeutischen Präparate können einerseits bei einer bereits bestehenden Krankheit und andererseits auch prophylaktisch gegeben werden. Beispielsweise kommen folgende Verabreichungsmöglichkeiten in Betracht:
a) orale Verabreichung etwa zur Behandlung des Gastrointestinattrakfes, Harntraktes, als Zusatz zu Rehydratationslösungen, als Prophylaktikum gegen Fehlbesiedlungen im Magen-Darmtrakt etc.,
b) topische Anwendungen etwa zur Behandlung des Rachenraums, Urogenitaltrakts etc., und
c) parenterale Anwendung.

Oligogalakturonide kommen als Teilstrukturen von Polysacchariden in einer großen Anzahl von Futtermittetn und Futtermittelhilfsstoffen vor und werden somit peroral eingenommen, d.h. sie sind nicht toxisch und eignen sich hervorragend für pharmazeutische Anwendungen.

Kohlenhydrate, analog den Rezeptoren für pathogene-Mikroorganismen sind bereits in Konzentrationen von 0,05 bis 0,1 % im Stande, die Adhärenz bakterieller Mikroorganismen in vitro zu blockieren.

Die Gewinnung der für die erfindungsgemäßen pharmazeutischen Präparate verwendeten Galakturonide kann aus in Pflanzen vorkommenden Polysacchariden erfolgen, z.B. aus Karotten, Zitrusfrüchten, Äpfeln, Quitten, Zuckerrüben, Kokosmilch und anderen Pflanzen oder Pflanzenprodukten.

Zur Gewinnung der Oligogalakturonide werden die Ausgangsmaterialien vorzugsweise zerkleinert und mit:heißem Wasser extrahiert. Die Extrakte können zuerst lyophilisiert oder auch direkt weiterverarbeitet werden. Die Weiterverarbeitung erfolgt vorzugsweise mit chromatographischen Methoden, durch die eine Abtrennung der Galakturonide von anderen Bestandteilen des Extrakts (z.B. anderen Sacchariden) möglich ist. Bevorzugte chromatographische Trennmethoden sind Gelchromatographie (z.B. BioGel® -, Sephacryl® -Trennmittel) oder/und Anionenaustauscherchromatographie (z.B. DEAE-Sephacel®⁻Trennmaterialien). Die erhaltenen Pektine können weiterhin zur Verringerung des Veresterungsgrades teilweise oder vollständig verseift oder/und zur Verringerung des Polymerisationsgrades durch Säurebehandlung bzw. enzymatisch hydrolysiert werden.

### Figurenbeschreibung

Abbildung 1 zeigt eine tägliche Aufzeichnung der Zahl der verendeten Tiere pro Gruppe;

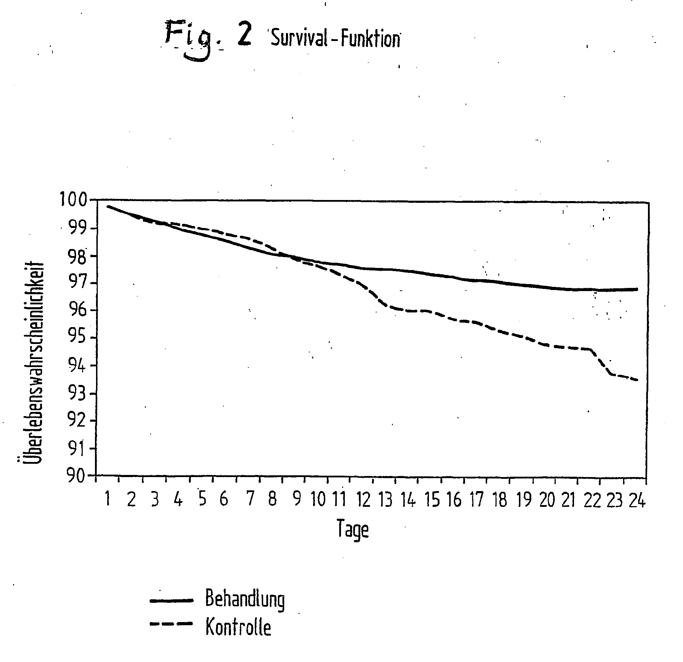

Abbildung 2 zeigt die Überlebenswahrscheinlichkeit als Survival-Funktion;
Abbildung 3 zeigt die E. coli Verschiebung in der Darmflora in den einzelnen Handlungsabschnitten.
Legende: hg = hochgradig, mg = mittelgradig, gg = geringgradig, v = vereinzelt; V = Vorbeobachtungszeit; n = 10, BV = Behandlungszeit Versuchsgruppe n = 20, BK = Behandlungszeit Kontrollgruppe n = 20.

Die Erfindung soll weiterhin durch die vorliegenden Beispiele erläutert werden.

### Beispiel 1

Frische Karotten werden gewaschen, geschält und geraspelt. Durch Extraktion mit siedendem Wasser und darauffolgende Lyophilisation werden die wasserlöslichen Polysaccharide gewonnen.

Es folgt eine erste gelchromatographische Trennstufe über eine Säule mit BioGel P2 und destilliertem Wasser als Eluent. Die Substanzen, die am Ausschlußvolumen dieser Säule eluieren (SF A) werden vereinigt und ein zweites Mal gelchromatographisch aufgetrennt. Als Trennmaterial dient Sephacryl S-3000HR und als Eluent nochmals destilliertes Wasser. Von den zwei Substanzpeaks, die nun erscheinen, wird der später eluierende gesammelt.

Der dritte Aufbereitungsschritt ist eine Ionenaustauscherchromatographie über DEAE-Sephacel mit einem Puffergradienten als Eluent (0,011 M bis 1 M Phosphatpuffer, pH 6,4). Die Fraktion, die mit höherer Molarität eluiert wird (SF II), wird gepoolt und weiter verarbeitet. Daraufhin erfolgt eine Hydrolyse mit HCl (pH 1,00, 37 °C, 45 min) und die Wiedergewinnung der Saccharide durch Alkoholfällung.

Das Hydrolysat wird auf gelchromatographischem Weg (Sephacryl S-2000HR, Eluent: destilliertes Wasser) in drei Substanzgruppen aufgetrennt, wobei die Kohlenhydrate mit dem geringsten Molekulargewicht (SF 2/3) gesammelt werden.

Die antiadhäsive Aktivität der Fraktionen, mit denen weitergearbeitet wird, übertrifft die der jeweils anfallenden Nebenprodukte.

Die Hemmintensität der einzelnen Substanzen steigt im Laufe der Aufarbeitung von 50 % in 0,7 %-iger Lösung (bei SF A) über 86,4 % in 0,005 %-iger Lösung (bei SF II) auf vollständige Hemmung ebenfalls in 0,005 %-iger Lösung (bei SF 2/3).

Dies deutet darauf hin, dass mit zunehmender Reinheit die zur Erzielung einer bestimmten Wirkung notwendige Konzentration deutlich geringer wird. Dies steht im Gegensatz zu im Handel befindlichen Pektinen, die in sehr hohen Konzentrationen eingesetzt werden müssen, um ähnliche Effekte erzielen zu können.

### Beispiel 2

### Verseifung:

11,3 g Zitruspektin, Fa. Grindstedt (Substanz A₀) werden in 1200 ml destilliertem Wasser gelöst, mit 480 ml 0,5 N NaOH versetzt und 15 min bei Raumtemperatur verseift. Die Lösung wird mit Ameisensäure konz. auf pH 4,0 eingestellt. Das verseifte Pektin wird mit dem doppelten Volumen Methanol gefällt, in 200 ml Wasser gelöst und die Fällung noch zwei Mal in gleicher Weise wiederholt. Der zuletzt gewonnene Niederschlag wird bei 50 °C getrocknet (Substanz A₁).
Auswaage: 7,15 g.

### Enzymatische Hydrolise:

Der getrocknete Niederschlag wird mit 100 ml 0,1 M Natriumhydrogencarbonat-Lösung gelöst, mit Ameisensäure (1 M) auf pH 4,5 eingestellt, mit 21 mg Pectinase 5S aus Aspergillus niger (Fa. Serva) versetzt und 60 min bei 60 °C inkubiert. Der Ansatz wird zur Enzymaktivierung kurze Zeit auf 80 °C erwärmt, abgekühlt und in oben beschriebener Weise mit Methanol gefällt. Durch Zentrifugation (20 Minuten, 4000 U/min) wird der Niederschlag gewonnen und getrocknet. Auswaage: 5 g (Substanz A₂).

Durch dünnschichtchromatographische Kontrolle wird überprüft, ob die Hydrolyse ausreichende Mengen an den gewünschten Oligogalakturoniden erzeugte.

### Dünnschichtchromatographiebedingungen:

| | |
|---|---|
| Stationäre Phase: | DC-Fertigplatten Kieselgel 60 (Fa. Merck) 10 cm x 20 cm |
| Fließmittel: | Ethanol: wässrige Essigsäure 25 mM 1:1 |
| Entwicklung: | bei 35 °C |
| Sprühreagenz: | 200 mg Naphthalin-1-3-diol in 50 ml Methanol plus 50 ml H₂SO₄ (20 % g/g) |

### Chromatographie:

1,5 g des Hydrolyseproduktes werden in 15 ml destilliertem Wasser gelöst und der chromatographischen Fraktionierung unterworfen.

| | |
|---|---|
| Säule: | Glassäule der Fa. Bio-Rad (2,5/40 cm) Bettvolumen 150 ml |
| Eluent: | Na-Formiatpuffer pH 4,7, Stufengradient je 182 ml 0,2, 0,3, 0,4, 0,5, 0,6, 0,7 M |
| Stationäre Phase: | Bio-Rad AGMP 1 Anionenaustauscherharz (mit Eluent äquilibriert) |
| Fließrate: | 3 ml/min |
| Fraktionsvol.: | 22 ml |

Die einzelnen Fraktionen werden mittels DC (Bedingungen siehe oben) auf ihre Zusammensetzung untersucht. Fraktionen gleicher Zusammensetzung werden vereinigt und mit dem zweifachen Volumen Aceton gefällt. Die auf diese Art und Weise gewonnenen Oligogalakturonide werden durch Zentrifugation (4000 U/min) gewonnen und in 20 ml destilliertem Wasser aufgenommen.

In die Lösung wird Bio-Rad AG 50 W X-8 Kationenaustauscher (H +) eingerührt, um die Galakturonide in die freie Säureform überzuführen. Nach Entfernen des Harzes und Lyophilisieren des Filtrats erhält man flockiges, weißes Pulver.

Die Ergebnisse der Hemmung sind in der folgenden Tabelle, 1 dargestellt.

**Tabelle 1**

| Substanz- bezeichnung | Charakterisierung | Konz. in % | Blockierung in % |
|---|---|---|---|
| A₀ | Pektin USP¹⁾ | 0,7 | 15,3 |
| A₁ | verseiftes Pektin | 0,2 | 10,0 |
| A₂ | partiell,hydrolysiertes A₁ | 0,05 | 30,2 |
| F₁ | neutrales Di- und Trisaccharid | 0,005 | 0,0 |
| F₂ | neutrales Trisaccharid | 0,005 | 0,0 |
| F₃ | neutrales Tetrasaccharid | 0,005 | 0,0 |
| F₄ | Trigalakturonid | 0,005 | 84,6 |
| F₅ | Tetragalacturonid mit Spuren Trigalacturonid | 0,005 | 58,6 |
| F₆ | Tetragalacturonid | 0,005 | 52,7 |
| F₇ | Penta-Hexagalacturonid | 0,005 | 23,9 |

| | | | |
|---|---|---|---|
| ¹⁾ Zitruspektin (F. Grindstedt) | | | |

### Beispiel 3

### Material und Methode

### Tierpopulation

2300 Tiere in einer Legehühnerfarm zeigten in den Wochen vor Beginn der Behandlungsstudie folgende klinische Symptome: die Hühner befanden sich in einem schlechten Allgemein- und Ernährungszustand, die Majorität der Hühner zeigten blutige Durchfälle und eine übelriechende Kloake. Die Legeleistung nahm kontinuierlich ab, die täglichen Tierverluste in der Herde beliefen sich im Mittel auf 5 Tiere pro Tag.

### Versuchsdesign

Die Tiere wurden für den Behandlungsversuch randomisiert in zwei annähernd gleich große Gruppen geteilt, von denen nur eine (die Therapiegruppe) erfindungsgemäß behandelt wurde. Alternierend stand den beiden Gruppen ein Freilandgehege zur Verfügung. Der Versuch gliedert sich prospektiv in drei Abschnitte:
1. Vorbeobachtungszeit: Die Vorbeobachtungszeit diente zur Beschreibung des Zustandes der Hühnerpopulation, der Ausgangsleistung und zum Vergleich zwischen den beiden Gruppen.
2. Behandlungszeitraum 1 Woche. In diesem Zeitraum wurde dem Futter für die Behandlungsgruppe die untersuchte Wirksubstanz beigemischt.
3. Nachbeobachtungszeit: 10 Tage zur Evaluierung der Langzeitwirkung der Oligogalakturonide.

### Futtermittel

Alle Hühner erhielten ein Standardfutter mit ... Mais ... In der Therapiegruppe wurden zusätzlich 1 % saure Oligogalakturonide beigemengt. Die übliche Futtermenge pro Huhn betrug 140 g/Tag. Die Hühner erhielten zusätzlich Wasser ad libitum. Die Behandlungsdauer betrug 7 Tage.

### Klinische Evaluierung:

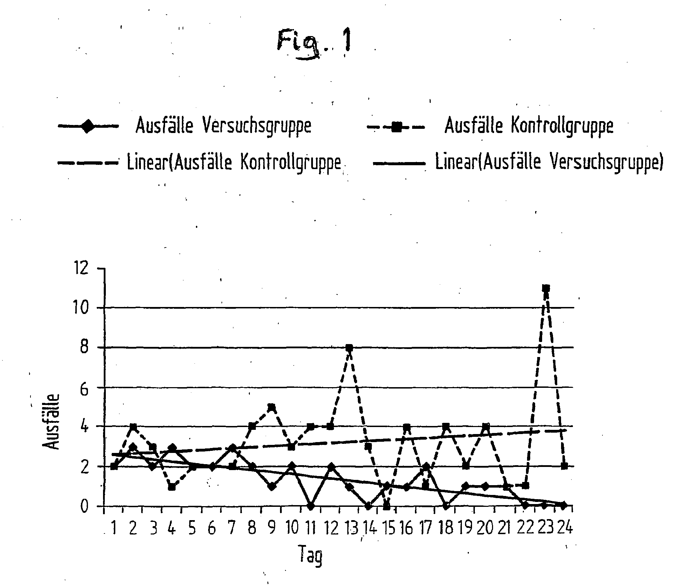

a) Beurteilung der Ausfälle: Es wurde eine tägliche Aufzeichnung der Zahl der verendeten Tiere pro Gruppe durchgeführt. (Abb. 1)
b) Pathologische Untersuchung: Am Ende der Vorbeobachtungszeit wurden 10 Tiere geschlachtet und auf pathologische Veränderungen der Organsysteme Vor allem des Darms und der Genitalorgane untersucht. Am Ende der Behandlungszeit und 10 Tage nach Ende der Behandlungszeit wurden je 20 Tiere in jeder Gruppe geschlachtet und auf die oben erwähnten pathologischen Organveränderungen untersucht.
c) Mikrobiologische Untersuchung: Von den oben beschriebenen Tieren wurden semiquantitative Untersuchungen der Stuhlflora durchgeführt. Die Untersuchungen erfolgten auf gramnegative Enterobacteriaceae mit Differenzierung zwischen E.coli und spezieller Berücksichtigung des hühnerpathogenen Stammes 078/K80 sowie Proteus spp. Des Weiteren wurden Enterokokken, Staphylokokken, aerobe Sporenbildner, Clostridium spp und Candida albicans differenziert. Eine semiquantitative Beurteilung erfolgte in
   vereinzelt = 10³
   geringgradig = 10⁵
   mittelgradig = 10⁷
   hochgradig = 10⁹ Besiedelung pro Gramm Stuhl.
d) Beurteilung der Leistungssteigerung
Die Legeleistung der Legehennen in der Gruppe der Oligosaccharidbehandelten Gruppe wurde mit der Legeleistung der unbehandelten Tiere verglichen. Täglich wurde die Eizahl pro Gruppe aufgezeichnet. Auch die Eiqualität, Schalendicke, Bruchfestigkeit, Blutbefleckung wurde beurteilt.

### Ergebnisse

### Klinische Evaluierung

Die beiden Gruppen unterschieden sich innerhalb von 2 bis 3 Tagen nach Beginn der Fütterung mit Oligogalakutroniden wesentlich in ihrem weiteren klinischen Verlauf.

### a) Anzahl der Ausfälle

Die Anzahl der Tierverluste in der Vorbeobachtungszeit, der Behandlungszeit und der Nachbeobachtungszeit ist in Tabelle 1 dargestellt. Es zeigte sich, dass in der Vorbeobachtungszeit von 5 Tagen und in den ersten 3 Tagen der Behandlung in beiden Gruppen ähnlich hohe Tierverluste zu beobachten waren. Ab dem 4. Behandlungstag kommt es zu einem deutlichen Auseinanderklaffen der Tierverluste: während in der Behandlungsgruppe zwischen dem 4. und 7. Behandlungstag 5 Tiere verendeten, kam es in der Kontrollgruppe im gleichen Zeitraum zu einem Verlust von 16 Tieren. Auch in der Nachbeobachtungszeit von 12 Tagen hielt dieser Effekt an: während in der Behandlungsgruppe 8 Tiere verendeten; kam es in der Kontrollgruppe im gleichen Zeitraum zu einem Verlust von 39 Tieren. Die Darstellung der Überlebenswahrscheinlichkeit der Tiere in beiden Gruppen ist als Survival-Function in Tabelle 2 dargestellt.

### b) Pathologische Untersuchungen

In der Vorbeobachtungszeit wurden bei 40 % der geschlachteten Tiere pathologische Veränderungen in Form von fibrinösen Serösitis, Peritonitis und Salpingitis beobachtet. Am Ende der Behandlungsperiode wurden in der Kontrollgruppe bei 35 % der Tiere in der Gruppe der behandelten Tiere bei nur mehr 10 % pathologische Veränderungen gefunden. 10 Tage nach Behandlungsende wurden in der Kontrollgruppe bei 45 %, in der Behandlungsgruppe bei 12 % pathologische Organveränderungen konstatiert. Dieser günstige Effekt blieb auch über eine Beobachtungszeit von 14 Tagen nach Absetzen der Galakturonide in der Nahrung erhalten.

Semiquantitative Untersuchungen der Stuhlflora ergaben eine substanzielle Abnahme der pathologischen Colifiora in der Gruppe der behandelten Tiere und eine weitgehende Regeneration der normalen Flora im Vergleich zur nicht behandelten Gruppe von Tieren. Auch die Legeleistung hatte sich in der Gruppe der behandelten Tiere im Beobachtungszeitraum um mindestens 5,8 % verbessert.

Oligogalakturonide eignen sich in der Behandlung schwerer bakterieller . Infektionen in der Tiermedizin als Ersatz von Antibiotika. Sie werden gut vertragen, sind nicht mit Resistenzentwicklung behaftet und besitzen einen nachhaltigen Effekt, der auch nach Absetzen der Zusatznahrung erhalten bleibt.

### c) Bakteriologische Untersuchungen

In der Vorbeobachtungszeit wurden von 10 Hühnern die Stuhlproben semiquantitativ beurteilt. Es zeigte sich, dass 8 der 10 Hühner hochgradig mit E.coli besiedelt, 1 Huhn hochgradig mit Proteus spp. besiedelt waren. 5 der 10 Hühner zeigten eine Besiedelung mit dem hühnerpathogenen Serotyp 078 K80.

In der Behandlungsphase kam es zu bemerkenswerten quantitativen Änderungen der Darmflora: Die normale Darmflora persisitierte in beiden Gruppen. Es kam jedoch zu einer deutlichen quantitativen Verschiebung der Coli-Flora während der Behandlung: 7 von 20 Hühnern in der Behandlungsgruppe zeigten eine hochgradige Besiedelung mit E.coli, 9 von 20 eine mittelgradie und 3 eine geringgradige Besiedelung. Bei 2 persistierte die Besiedelung mit 078 K80. In der Kontrollgruppe hingegen zeigten 16 von 20 Hühnern eine hochgradige und 4 von 20 Hühnern eine mittelgradige Besiedelung mit E.coli. Bei 4 von 20 bestand eine hochgradige Besiedelung mit Proteus spp. 5 von 20 Hühnern zeigten eine persistierende Besiedelung mit E.coli 078 K80.

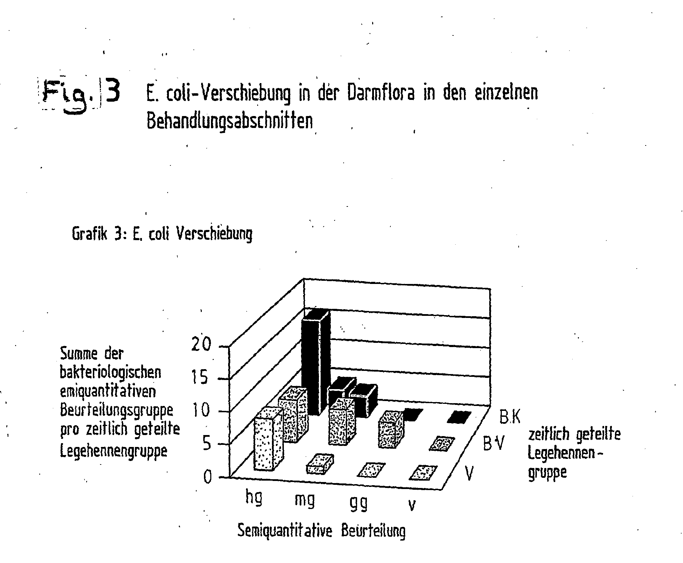

Abb.3 zeigt die Änderung der Darmflora in den einzelnen Behandlungsabschnitten. In der Gruppe der Hühner, die Oligogalakturonide in der Nahrung erhielten, blieb die physiologische Zusammensetzung der Flora erhalten.

### Beurteilung der Legeleistung:

Der Unterschied in der Legeleistung zwischen den mit Oligosacchariden behandelten Hühner und den Hühnern in der Kontrollgruppe nach Ausgleich der verendeten Hühner betrug im Therapiezeitraum 3,79 %, in der Nachbeobachtungszeit 4,2 %. Das ergibt eine Gesamtdifferenz von 4,08 % (=47 Eier/Tag) zugunsten der Versuchsgruppe. Es ergibt sich hiermit ein deutlich positiver. Wert-zugunsten der mit Galakturoniden behandleten Hühner.

### Literatur

(1) Heider G., Monreal G. (Hrsg.) (1992) Krankheiten des Wirtschaftsgeflügels, Band II, Spezieller Teil 2, Gustav Fischer Verlag, Jena Stuttgart, S. 103/104;
(2) Schwarz E., pers. Mitteilung;
(3) Tauxe R.V. (1997), Emerging Foodborne Diseases: An Evolving Public Health Challange, Emerg. Inf. Dis. 3 (4):425-434;
(4) Christ W. (1999) Antibiotika - Entwicklungen und Trends, MMP 3: 72-78;
(5) The Medical Impact of the Use of Antimicrobials in Food Animals, Report of a WHO Meeting, Berlin, Deutschland, 13.-17. Oktober 1997;
(6) McDonald L.C., Kuehnert M.J., Tenover F.C., Jarvis W.R., (1997), Vancomycin-Resistent Enterococci Outside the Health-Care Setting: Prevalence, Sources and Public Health Implications; Emerg. Inf. Dis. 3(3):311-317;
(7) Nataro J.P., Kaper J.B. (1998) Diarrheagenic Escherichia coli, CMR 11 (1): 142-201;
(8) Schmitt C.K., Meysick K.C., O'Brien A.D., (1999) Bacterial Toxins: Friends or Foes?, Emerg. Inf. Dis. 5(2):224-234;
(9) Zopf D., Roth S. (1996) Oligosaccharide anti-infective agents, The Lancet 347: 1017-1021;
(10) Smith H.W., Linggood M.A., (1971) Observation on the pathogenic properties of the K 88; Hly and Ent plasmid of E.coli with particular reference to porcine diarrhea, J. Med. Microbiol. 4: 467-485;
(11) Leffler H., Svanborg Eden C. (1980), Chemical identification of a glycoshingolipid receptor of Escherichia coli atteaching to human urinary tract epithelial cells and aggluttinating human erythrocytes, FEMS Microbiol. Letters 8: 127-134;
(12) Guggenbichler J.P., De Bettignies-Dutz A., Meißner P., Schellmoser S., Jurenitsch J. (1997) Acidic ofigosaccharides form natural sources block adherence of Escherichia coli on uroepithelial cells,; Pharm. Pharmacol. Lett. 7:35-38;
(13) Cravioto A., Tello. A., Villafan H., Ruiz J., del Vedovo S., Neeser J.-R. (1991), Inhibiton of Localized Adhesion of Enteropathogenic Escherichia coli to HEp-2 Cells by Immunoglobulin and Oligosaccharide Fractions of Human Colostrum and Breast Milk, J. Inf. Dis. 163: 1247-1255;
(14) Gilboa-Garber N., Garber N., Microbial Lectins: In Allen H.J., Kisailus E.C. (Hrsg.) (1992) Glycoconjugates, Marcel Dekker Inc., New York, Basel, Hong Kong, S. 541-591;
(15) Schellmoser S., Follrich B., Kastner U., Jurenitsch J., Guggenbichier J.P. (1997) Acidic Oligosaccharides from Natural Sources Block Adherence of Escherichia coli on Gastrointestinal Epithelia and Uroepithelia, 15^{th} Annual Meeting of the European Society for Paediatric Infectious Diseases, 21.-23. Mai 1997, Paris, ESPID 97, Abstract Book S. 127;
(16) Devriese L.A., leven M., Goossens H., Vandamme P., Pot B., Hommez J., Haesebrouk F. (1996), Presence of vancomycin-resistant enterococci in farm animals, Antimicrob. Agents-Chemother 40(10): 2285-2287;
(17) Aarestrup F.M., Ahrens P., Madsen M., Pallensen L.V., Poulsen R.L., Westh H. (1996) Glycopeptide susceptibility among Danish Enterococcus faecium and Enterococcus faecalis isolates of animal and human origin and PCR identification of genes within the VanA cluster, Antimicrob. Agents Chemother. 40(8): 1937-1940;
(18) Farm animals as a putative reservoir for vancomycin-resistant enterococcal infection in man, J. Antimicrob. Chemother. 34(4): 507-514;
(19) Svanborg Eden C., Andersson B., Aninansson G., Lindstedt R., de Man P., Nielsen A., Leffler H., Wold A. (1990) Inhibition of bacterial attachment: examples from the urinary and respiratory tracts, Curr. Top. Microb. Immunol. 151:166-184.

## Patentansprüche

1. Verfahren zur Herstellung eines pharmazeutischen Präparates zur Blockierung der Anlagerung von Keimen an Vogelzellen,
**dadurch gekennzeichnet,**
**dass** man ein oder mehrere Oligogalakturonide mit einem Polymerisationsgrad ≥ 2 und einem Veresterungsgrad < 20 % als Wirkstoff, gegebenenfalls zusammen mit pharmazeutisch üblichen Träger-, Verdünnungs-, Hilfs- und Füllstoffen in eine zur Verabreichung geeignete Form bringt.

2. Verfahren nach Anspruch 1,
**dadurch gekennzeichnet,**
**dass** der Veresterungsgrad des Oligogalakturonids < 10 % ist.

3. Verfahren nach Anspruch 1 oder 2,
**dadurch gekennzeichnet,**
**dass** der Veresterungsgrad des Oligogalakturonids < 5 % ist.

4. Verfahren nach einem der Ansprüche 1 bis 3,
**dadurch gekennzeichnet,**
**dass** der Polymerisationsgrad des Oligogalakturonids 2 bis 7 ist.

5. Verfahren nach einem der Ansprüche 1 bis 4,
**dadurch gekennzeichnet,**
**dass** der Polymerisationsgrad des Oligogalakturonids 2 bis 6 ist.

6. Verfahren nach einem, der Ansprüche 1 bis 5,
**dadurch gekennzeichnet,**
**dass** der Polymerisationsgrad des Oligogalakturonids 2 bis 5 ist.

7. Verfahren nach einem der Ansprüche 1 bis 6,
**dadurch gekennzeichnet**
**dass** der Polymerisationsgrad des Oligogälakturonids 2 bis 4 ist.

8. Verfahren nach einem der Ansprüche 1 bis 7,
**dadurch gekennzeichnet,**
**dass** der Polymerisationsgrad des Oligogalakturonids 2 bis 3 ist.

9. Verfahren nach einem der Ansprüche 1 bis 8,
**dadurch gekennzeichnet,**
**dass** der Polymerisationsgrad des Oligogalakturonids 2 ist.

10. Verfahren nach einem der Ansprüche 1 bis 9 für die Herstellung eines pharmazeutischen Präparates zur Behandlung von Infektionen des Gastrointestinaltraktes, des Blutsystems, der Atemwege, Des Urogenitaltrakts oder/und des Rachenraums von Vögeln.

## Claims

1. Process for the production of a pharmaceutical preparation for blocking the attachment of germs to bird cells,
**characterized in that**
one or more oligogalacturonides having a degree of polymerization of ≥ 2 and a degree of esterification of < 20 % serving as the active substance optionally together with common pharmaceutical carrier substances, diluents, adjuvants and fillers are brought into a form suitable for administration.

2. Process according to claim 1,
**characterized in that**
the degree of esterification of the oligogalacturonide is < 10 %.

3. Process according to claim 1 or 2,
**characterized in that**
the degree of esterification of the oligogalacturonide is < 5 %.

4. Process according to one of the claims 1 to 3,
**characterized in that**
the degree of polymerization of the oligogalacturonide is 2 to 7.

5. Process according to one of the claims 1 to 4,
**characterized in that**
the degree of polymerization of the oligogalacturonide is 2 to 6.

6. Process according to one of the claims 1 to 5,
**characterized in that**
the degree of polymerization of the oligogalacturonide is 2 to 5.

7. Process according to one of the claims 1 to 6,
**characterized in that**
the degree of polymerization of the oligogalacturonide is 2 to 4.

8. Process according to one of the claims 1 to 7,
**characterized in that**
the degree of polymerization of the oligogalacturonide is 2 to 3.

9. Process according to one of the claims 1 to 8,
**characterized in that**
the degree of polymerization of the oligogalacturonide is 2.

10. Process according to one of the claims 1 to 9 for the production of a pharmaceutical preparation for the treatment of infections of the gastrointestinal tract, the blood system, the respiratory tract, the urogenital tract or/and the pharyngeal cavity of birds.

## Revendications

1. Procédé pour la production d'une préparation pharmaceutique pour bloquer le dépôt de germes sur des cellules d'oiseau, **caractérisé en ce qu'**on met sous une forme appropriée à l'administration un ou plusieurs oligogalacturonides ayant un degré de polymérisation ≥ 2 et un degré d'estérification < 20 % comme principe actif, éventuellement avec des supports, diluants, adjuvants et charges pharmaceutiquement habituels.

2. Procédé selon la revendication 1 **caractérisé en ce que** le degré d'estérification de l'oligogalacturonide est < 10 %.

3. Procédé selon la revendication 1 ou 2 **caractérisé en ce que** le degré d'estérification de l'oligogalacturonide est < 5 %.

4. Procédé selon l'une des revendications 1 à 3 **caractérisé en ce que** le degré de polymérisation de l'oligogalacturonide ce 2 à 7.

5. Procédé selon l'une des revendications 1 à 4 **caractérisé en ce que** le degré de polymérisation de l'oligogalacturonide est 2 à 6.

6. Procédé selon l'une des revendications 1 à 5 **caractérisé en ce que** le degré de polymérisation de l'oligogalacturonide est 2 à 5.

7. Procédé selon l'une des revendications 1 à 6 **caractérisé en ce que** le degré de polymérisation de l'oligogalacturonide est 2 à 4.

8. Procédé selon l'une des revendications 1 à 7 **caractérisé en ce que** le degré de polymérisation de l'oligogalacturonide est 2 à 3.

9. Procédé selon l'une des revendications 1 à 8 **caractérisé en ce que** le degré de polymérisation de l'oligogalacturonide est 2.

10. Procédé selon l'une des revendications 1 à 9 pour la production d'une préparation pharmaceutique pour le traitement d'infections des voies gastro-intestinales, du système sanguin, des voies respiratoires, des voies urogénitales et/ou de la cavité du pharynx d'oiseaux.
